(19) Europäisches Patentamt
European Patent Office
Office européen des brevets

(11) Veröffentlichungsnummer: **0 107 009**
**A2**

(12) # EUROPÄISCHE PATENTANMELDUNG

(21) Anmeldenummer: **83109051.9**

(22) Anmeldetag: **14.09.83**

(51) Int. Cl.³: **A 01 N 25/04**

(30) Priorität: **25.09.82 DE 3235612**

(43) Veröffentlichungstag der Anmeldung: **02.05.84**
**Patentblatt 84/18**

(84) Benannte Vertragsstaaten: **AT BE CH DE FR GB IT LI NL**

(71) Anmelder: **BAYER AG, Konzernverwaltung RP**
**Patentabteilung, D-5090 Leverkusen 1 Bayerwerk (DE)**

(72) Erfinder: **Hausmann, Heinz, Dr., Dierath 5,**
**D-5653 Leichlingen (DE)**
Erfinder: **Niessen, Heinz-Josef, Dr., Pannenberg 73,**
**D-5060 Bergisch-Gladbach 2 (DE)**
Erfinder: **Horstmann, Heinz-Otto, Dr., Löhe 42,**
**D-5060 Bergisch-Gladbach 1 (DE)**

(54) **Mikroemulsionen.**

(57) Wäßrige Mikroemulsionen, die

0,1 bis 80 Gew.-% mindestens eines in Wasser wenig löslichen agrochemischen Wirkstoffes, eines Wirkstoffes zur Bekämpfung von Schädlingen im Haushalts- und Hygiene-Bereich und/oder eines pharmakologisch wirksamen Stoffes,

1 bis 20 Gew.-% eines Emulgatorgemisches aus

a) mindestens einem Alkylaryl-polyglykolether der Formel

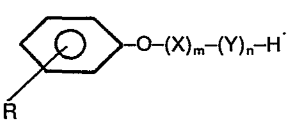 (I)

in welcher

R, X, Y, m und n die in der Beschreibung angegebene Bedeutung haben,

und mindestens einem Alkylarylsulfonsäure-Salz, oder

b) mindestens einem Alkylaryl-polyglykolether der Formel

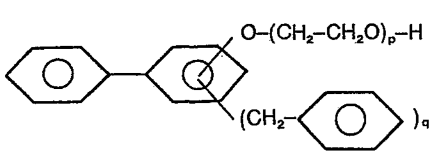 (III)

in welcher

p und q die in der Beschreibung angegebene Bedeutung haben,

und mindestens einem Alkylarylsulfonsäure-Salz

sowie Wasser und

gegebenenfalls 1 bis 30 Gew.-% mindestens eines mit Wasser wenig mischbaren organischen Lösungsmittels und/oder eines Solubilisators sowie

gegebenenfalls 0,05 bis 15 Gew.-% an Zusatzstoffen enthalten,

wobei die Summe der Komponenten jeweils 100 Gew.-% beträgt,

ein Verfahren zur Herstellung dieser wäßrigen Mikroemulsionen und deren Verwendung.

EP 0 107 009 A2

BAYER AKTIENGESELLSCHAFT 5090 Leverkusen, Bayerwerk

Zentralbereich
Patente, Marken und Lizenzen Dü/ABc 25.09.82
V

## Mikroemulsionen

Die vorliegende Erfindung betrifft wäßrige Mikroemulsionen von agrochemischen Wirkstoffen, Wirkstoffen zur Bekämpfung von Schädlingen im Haushalts- und Hygiene-Bereich und/oder pharmakologisch wirksamen Stoffen. Die Erfindung betrifft außerdem ein Verfahren zur Herstellung dieser Mikroemulsionen sowie deren Verwendung.

Es sind bereits Öl-in-Wasser-Emulsionen von zahlreichen in Wasser wenig löslichen agrochemischen Wirkstoffen bekannt, welche neben den Wirkstoffen jeweils noch entweder einen oberflächenaktiven Stoff und ein Verdickungsmittel oder aber eine relativ große Menge an oberflächenaktiven Stoffen enthalten (vgl. DE-A 30 09 944, DE-A 30 11 611 und JP-A 122 628-77). Dieser Zusatz an Verdickungsmittel bzw. an großen Tensidmengen ist mit zusätzlichen Kosten verbunden und stellt somit einen gravierenden Nachteil der bekannten Öl-in-Wasser-Emulsionen dar. Hinzu kommt, daß die bisher beschriebene Herstellung von derartigen Emulsionen nicht allgemein anwendbar ist. Im wesentlichen lassen sich danach nämlich nur solche in Wasser wenig löslichen Wirkstoffe emulgieren, die bei Raumtemperatur flüssig sind oder

zumindest einen sehr niedrigen Schmelzpunkt aufweisen. Nachteilig ist auch, daß die bekannten Öl-in-Wasser-Emulsionen häufig nicht ausreichend kältestabil sind, und daß in manchen Fällen eine Zwangsemulgierung mit Hilfe von Homogenisatoren erforderlich ist.

Es wurden nun wäßrige Mikroemulsionen gefunden, die

- 0,1 bis 80 Gew.-% mindestens eines in Wasser wenig löslichen agrochemischen Wirkstoffes, eines Wirkstoffes zur Bekämpfung von Schädlingen im Haushalts- und Hygiene-Bereich und/oder eines pharmakologisch wirksamen Stoffes,

- 1 bis 20 Gew.-% eines Emulgatorgemisches aus

  a) mindestens einem Alkylaryl-polyglykolether der Formel

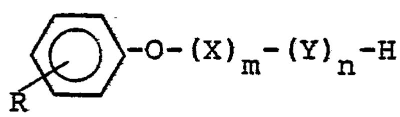

(I)

in welcher

R für Alkyl mit 8 bis 20 Kohlenstoffatomen steht,

X und Y jeweils für eine $-CH_2-CH_2-O-$,

$-CH_2-\underset{CH_3}{\underset{|}{C}}H-O-$ oder $-\underset{CH_3}{\underset{|}{C}}H-CH_2-O-$Gruppe

stehen, wobei jedoch X und Y nicht gleichzeitig für eine Oxyethylen- oder Oxypropylen-Einheit stehen,

m für Zahlen von 10 bis 45 steht und

n für Zahlen von 10 bis 45 steht,

und

mindestens einem Alkylarylsulfonsäure-Salz der Formel

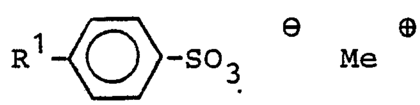

(II)

in welcher

$R^1$ für Alkyl mit 8 bis 35 Kohlenstoffatomen steht und

$Me^{\oplus}$ für ein Alkalimetallkation, ein Äquivalent eines Erdalkalimetallkations oder für ein Kation der Formel

$$\left[ R' - \overset{R''}{\underset{R^{IV}}{N}} - R''' \right]^{\oplus}$$

steht, worin

R', R", R"' und $R^{IV}$ unabhängig voneinander für Wasserstoff, Alkyl mit 1 bis 4 Kohlenstoffatomen oder Hydroxyalkyl mit 1 bis 4 Kohlenstoffatomen stehen,

oder

b) mindestens einem Alkylarylpolyglykolether der Formel

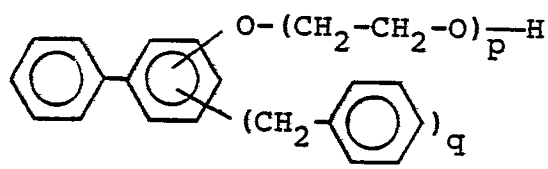

(III)

in welcher

p für Zahlen von 5 bis 20 steht und

q für Zahlen von 1 bis 3 steht,

und mindestens einem Alkylarylsulfonsäure-Salz der Formel

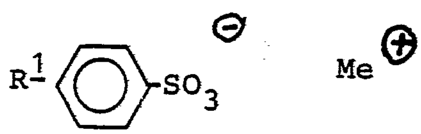

(II)

in welcher

$R^1$ und $Me^{\oplus}$ die oben angegebene Bedeutung haben,

- sowie Wasser und

- gegebenenfalls 1 bis 30 Gew.-% mindestens eines mit Wasser wenig mischbaren organischen Lösungsmittels und/oder eines Solubilisators sowie

- gegebenenfalls 0,05 bis 15 Gew.-% an Zusatzstoffen

enthalten, wobei die Summe der Komponenten jeweils 100 Gew.-% beträgt.

Weiterhin wurde gefunden, daß sich die erfindungsgemäßen Mikroemulsionen dadurch herstellen lassen, daß man eine homogene Mischung aus

- mindestens einem in Wasser wenig löslichen agrochemischen Wirkstoff, einem Wirkstoff zur Bekämpfung von Schädlingen im Haushalts- und Hygiene-Bereich und/oder einem pharmakologisch wirksamen Stoff,

- einem Emulgatorgemisch aus

  a) mindestens einem Alkylaryl-polyglykolether der Formel

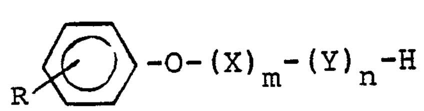

(I)

in welcher

R, X, Y, m und n die oben angegebene Bedeutung haben,

und

mindestens einem Alkylarylsulfonsäure-Salz der Formel

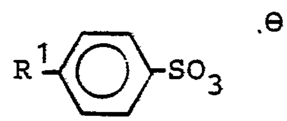 $Me^{\oplus}$ (II)

in welcher

$R^1$ und $Me^{\oplus}$ die oben angegebene Bedeutung haben,

oder

b) mindestens einem Alkylaryl-polyglykolether der Formel

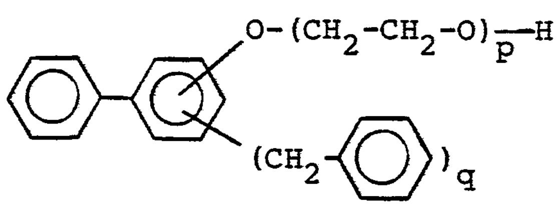 (III)

in welcher

p und q die oben angegebene Bedeutung haben,

und

mindestens einem Alkylaryl-sulfonsäure-Salz der Formel

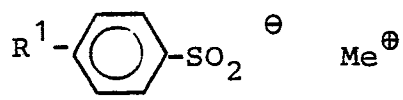 (II)

in welcher

$R^1$ und $Me^{\oplus}$ die oben angegebene Bedeutung haben,

- gegebenenfalls mindestens einem mit Wasser wenig mischbaren organischen Lösungsmittel und/oder einem Solubilisator sowie

- gegebenenfalls Zusatzstoffen

unter Rühren in gegebenenfalls Zusatzstoffe enthaltendes Wasser gibt.

Schließlich wurde gefunden, daß sich die erfindungsgemäßen Mikroemulsionen, - je nach den enthaltenen Wirkstoffen -, für verschiedene Zwecke in der Landwirtschaft und im Gartenbau, im Haushalts- und Hygiene-Bereich oder im medizinischen Bereich verwenden lassen.

Es ist als äußerst überraschend zu bezeichnen, daß die erfindungsgemäßen Mikroemulsionen stabil sind, denn auf Grund des bekannten Standes der Technik war zu erwarten, daß erartige Emulsionen, die keine Verdickungsmittel und auch nur einen geringen Tensidanteil aufweisen, nicht über längere Zeit haltbar sind. So geht aus der DE-A 30 09 944 und der DE-A 30 11 611 hervor, daß die dort beschriebenen Öl-in-Wasser-Emulsionen zwingend ein Verdickungsmittel als Stabilisator enthalten. Die in der JP-A 122 628-77 offenbarten Emulsionen weisen einen im Verhältnis zur Wirkstoffmenge sehr hohen Tensidanteil auf. Die hervorragende Beständigkeit der erfindungsgemäßen Mikroemulsionen konnte daher nicht vorausgesehen werden.

Die erfindungsgemäßen Mikroemulsionen zeichnen sich durch eine Reihe von Vorteilen aus. So erübrigt sich bei ihrer Herstellung ein kostspieliger Zusatz von Verdickungsmitteln oder großen Mengen an Emulgatoren. Weiterhin enthalten diese Emulsionen entweder gar keine, oder aber nur eine extrem geringe Menge an organischen Lösungsmitteln. Sie sind daher nicht brennbar, frei oder zumindest nahezu frei von Geruchsbelästigungen durch organische Lösungsmittel und weisen eine geringere Toxizität bzw. Phytotoxizität auf als entsprechende Formulierungen, die organische Lösungsmittel in den sonst üblichen Konzentrationen enthalten. Darüber hinaus sind die erfindungsgemäßen Mikroemulsionen unter den in der Praxis herrschenden Bedingungen stabil. Bei Langzeit-Lagerungen bleiben diese Emulsionen sowohl bei Temperaturen von 50°C als auch

bei tiefen Temperaturen unverändert. Schließlich lassen sich die erfindungsgemäßen Mikroemulsionen in einfacher Weise herstellen. Eine Zwangsemulgierung mit Hilfe von Homogenisatoren ist nicht erforderlich. Außerdem besteht ein ganz erheblicher Vorteil darin, daß sich bei Raumtemperatur feste bzw. flüssige, in Wasser wenig lösliche Wirkstoffe gleichermaßen gut emulgieren lassen. Weiterhin besitzen die erfindungsgemäßen Mikroemulsionen eine relativ geringe Viskosität, so daß eine genaue Dosierung keine Schwierigkeiten bereitet. Schließlich handelt es sich bei den erfindungsgemäßen Mikroemulsionen um völlig transparente Zubereitungen, die sich vor der Anwendung problemlos in jedem beliebigen Verhältnis mit Wasser verdünnen lassen, wobei stabile, spritzfähige Formulierungen entstehen.

Die erfindungsgemäßen Mikroemulsionen enthalten mindestens einen in Wasser wenig löslichen agrochemischen Wirkstoff, einen Wirkstoff zur Bekämpfung von Schädlingen im Haushalts- und Hygiene-Bereich und/oder einen pharmakologisch wirksamen Stoff. Diese Wirkstoffe liegen in der Ölphase im flüssigen Zustand vor.

Als Wirkstoffe kommen sowohl solche Substanzen in Frage, die bei Raumtemperatur flüssig sind, als auch solche, die bei Raumtemperatur fest sind. Voraussetzung für flüssige Wirkstoffe ist lediglich, daß sie in Wasser schwer löslich sind. Unter derartigen Stoffen sind hierbei Substanzen zu verstehen, die in Wasser bei 20°C zu maximal 0,5 Gew.-% löslich sind. Feste Wirkstoffe

müssen darüber hinaus jedoch in einem mit Wasser wenig mischbaren organischen Lösungsmittel und/oder einem Solubilisator hinreichend löslich sein.

Unter agrochemischen Stoffen sind im vorliegenden Fall alle üblicherweise im Pflanzenschutz verwendbaren Wirkstoffe zu verstehen. Hierzu gehören zum Beispiel Insektizide, Akarizide, Nematizide, Fungizide, Herbizide, Wachstumsregulatoren und Düngemittel. Als Beispiele für derartige Wirkstoffe seien im einzelnen genannt:

O,O-Diethyl-O-(4-nitro-phenyl)-thiono-phosphorsäureester
O,O-Dimethyl-O-(4-nitro-phenyl)-thiono-phosphorsäureester
O-(Ethyl)-O-(4-methylthio-phenyl)-S-propyl-dithiophosphat
(O,O-Diethylthionophosphoryl)-α-oximino-phenylessigsäurenitril
2-Isopropoxy-phenyl-N-methylcarbamat
3-Methylthio-4-amino-6-tert.-butyl-1,2,4-triazin-5-on
3-Methylthio-4-isobutylidenamino-6-tert.-butyl-1,2,4-triazin-5-on
2-Chlor-4-ethylamino-6-isopropylamino-1,3,5-triazin
2,3-Dihydro-2,2-dimethyl-7-benzofuranyl-methyl-carbamat
3,5-Dimethyl-4-methylthiophenyl-N-methyl-carbamat
O,O-Diethyl-O-(3-chlor-4-methyl-7-cumarinyl)-thiophosphat
6,7,8,9,10,10-Hexachlor-1,5,5A,6,9,9A-hexahydro-6,9-methan-2,3,4-benzo-dioxathiepin-3-oxid

1,4,5,6,7,8,8-Heptachlor-4,7-endo-methylen-3A,4,7,7A-tetrahydroinden

2-(2-Furyl)-benzimidazol

5-Amino-1-bis-(dimethylamido)-phosphoryl-3-phenyl-1,2,4-triazol

4-Hydroxy-3-(1,2,3,4-tetrahydro-1-naphthyl)-cumarin

S-[1,2-Bis-(ethoxycarbonyl)-ethyl]-O,O-dimethyl-dithiophosphorsäureester

O,O-Dimethyl-O-(4-methylmercapto-3-methyl-phenyl)-thionophosphorsäureester

O-Ethyl-O-(2-isospropyloxycarbonyl-phenyl)-N-isopropyl-thionophosphorsäureesteramid

1-(4-Chlorphenoxy)-3,3-dimethyl-1-(1,2,4-triazol-1-yl)-2-butanon

(S)-α-Cyano-3-phenoxybenzyl(1R)-cis-3-(2,2-dibromvinyl)-2,2-dimethylcyclopropancarboxylat

2,2-Dimethyl-3-(ß,ß-dichlorvinyl)-cyclopropancarbonsäure-α-cyano-3-phenoxy-4-fluor-benzylester.

Unter Wirkstoffen zur Bekämpfung von Schädlingen im Haushalts- und Hygiene-Breich sind im vorliegenden Fall alle üblichen für derartige Indikationen in Frage kommenden in Wasser wenig lösliche Wirkstoffe zu verstehen. Als Beispiele für derartige Wirkstoffe seien im einzelnen genannt:

2-Isopropoxy-phenyl-N-methylcarbamat

O,O-Diethyl-O-(4-nitro-phenyl)-thionophosphorsäureester

O,O-Dimethyl-O-(4-nitro-phenyl)-thionophosphorsäureester

S-[1,2-Bis-(ethoxycarbonyl)-ethyl]-O,O-dimethyl-dithiophosphorsäureester

O,O-Dimethyl-O-(3-methyl-4-nitro-phenyl)-thionophosphorsäureester
O,O-Dimethyl-O-(4-methylmercapto-3-methyl-phenyl)-thionophosphorsäureester
γ-Hexachlorcyclohexan
(Cyclohex-1-en-1,2-dicarboximidomethyl)-2,2-dimethyl-3-(2-methylpropenyl)-cyclopropancarboxylat

Unter pharmakologisch wirksamen Stoffen sind im vorliegenden Fall vorzugsweise im veterinärmedizinischen Bereich einsetzbare, in Wasser wenig lösliche Stoffe zu verstehen. Als Beispiel für derartige Wirkstoffe sei genannt:

2,2-Dimethyl-3-[ß-(p-chlorphenyl)-ß-chlorvinyl]-cyclopropancarbonsäure-α-cyano-3-phenoxy-4-fluor-benzylester.

Die in den erfindungsgemäßen Mikroemulsionen enthaltenen Emulgator-Gemische bestehen entweder aus mindestens einem Alkylaryl-polyglykolether der Formel (I) und mindestens einem Alkylarylsulfonsäure-Salz der Formel (II) oder aus mindestens einem Alkylaryl-polyglykolether der Formel (III) und mindestens einem Alkylarylsulfonsäure-Salz der Formel (II).

Die Alkylaryl-polyglykolether der Formel (I) sind durch die angegebene Formel allgemein definiert. In dieser Formel steht R vorzugsweise für Alkyl mit 10 bis 18 Kohlenstoffatomen. X und Y stehen jeweils für eine

$-CH_2-CH_2-O-$, $-CH_2-\underset{CH_3}{\underset{|}{CH}}-O-$ oder $-\underset{CH_3}{\underset{|}{CH}}-CH_2-O-$Gruppe, wobei jedoch X und Y nicht gleichzeitig für eine Oxyethylen- oder Oxypropylen-Einheit stehen. Der Index m steht vorzugsweise für Zahlen von 12 bis 30, und der Index n steht vorzugsweise für Zahlen von 12 bis 40. Die Zahlen für die Indices m und n stellen Durchschnittswerte dar.

Die in der Praxis verwendeten Emulgatoren dieses Tpys sind im allgemeinen Gemische aus mehreren Verbindungen der Formel (I). Insbesondere handelt es sich hierbei um Gemische aus Stoffen der Formel (I), die sich durch die Zahl der Oxethylen- bzw. Oxypropylen-Einheiten unterscheiden. Dadurch errechnen sich für die Indices m und n auch gebrochene Zahlen als Mittelwerte. Beispielsweise erwähnt seien Substanzen, für die sich die folgenden durchschnittlichen Zusammensetzungen ergeben:

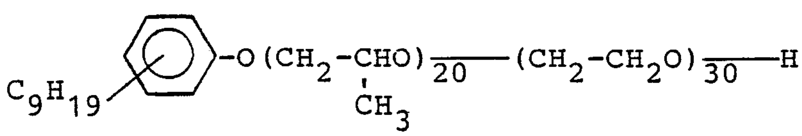

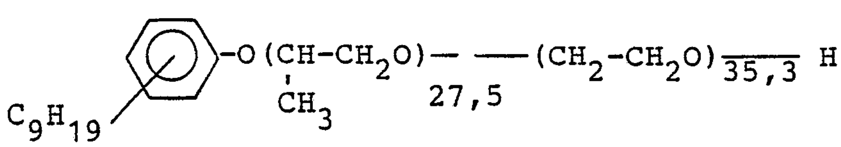

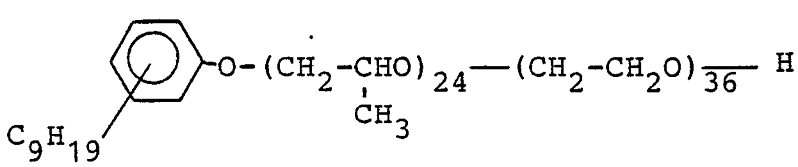

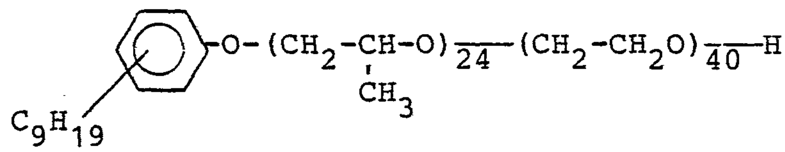

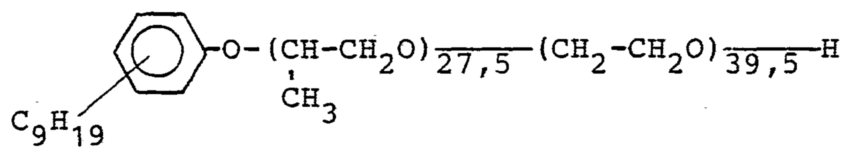

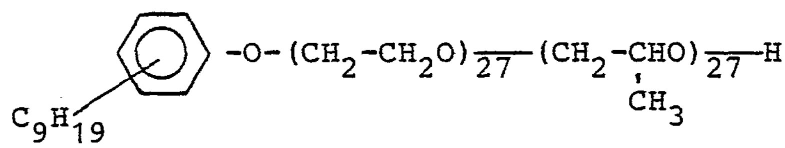

Die Alkylaryl-polyglykolether der Formel (I) sind bekannt.

Die in der erfindungsgemäßen Mikroemulsionen enthaltenen Alkylaryl-sulfonsäure-Salze sind durch die Formel (II) allgemein definiert. In dieser Formel steht $R^1$ vorzugsweise für geradkettiges oder verzweigtes Alkyl mit 9 bis 30 Kohlenstoffatomen. $Me^{\oplus}$ steht vorzugsweise für ein Natriumkation, für ein Äquivalent eines Calciumkations oder für ein Kation der Formel

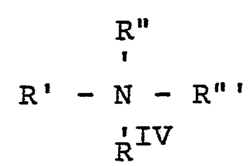

worin

R', R", R"' und $R^{IV}$ unabhängig voneinander vorzugsweise für Wasserstoff, Alkyl mit 1 oder 2 Kohlenstoffatomen oder Hydroxyalkyl mit 1 bis 2 Kohlenstoffatomen stehen.

Als Beispiele für Alkylaryl-sulfonsäure-Salze der Formel (II) seien im einzelnen genannt:

4-(n-Nonyl)-phenyl-sulfonsäure-Natriumsalz
4-(n-Dodecyl)-phenyl-sulfonsäure-Calciumsalz
4-(Tetrapropylen)-phenyl-sulfonsäure-Natriumsalz
4-(n-Nonyl)-phenyl-sulfonsäure-Calciumsalz
4-(i-Dodecyl)-phenyl-sulfonsäure-Ammoniumsalz
4-(n-Dodecyl)-phenyl-sulfonsäure-(2-hydroxyethyl)-ammoniumsalz.

Die Alkylaryl-sulfonsäure-Salze der Formel (II) sind bekannt. Sie werden im allgemeinen als 50-75 %ige Lösungen in organischen Lösungsmitteln, z.B. n- oder i-Butanol oder Benzylalkohol, eingesetzt, können grundsätzlich aber auch ohne Lösungsmittel verwendet werden.

Die Alkylaryl-polyglykolether der Formel (III) sind durch die angegebe Formel allgemein definiert. In dieser Formel steht der Index p vorzugsweise für Zahlen von 8 bis 18, und der Index q steht vorzugsweise für Zahlen von 1 bis 2. Die Zahlen für die Indices p und q stellen Durchschnittswerte dar.

Die in der Praxis verwendeten Emulgatoren dieses Types sind im allgemeinen Gemische aus mehreren Verbindungen der Formel (III). Insbesondere handelt es sich hierbei um Gemische aus Stoffen der Formel (III), die sich durch die Zahl der Oxyethylen-Einheiten bzw. durch den Substitutionsgrad am Phenylrest unterscheiden. Dadurch können sich für die Indices p und q auch gebrochene Zahlen als Mittelwerte errechnen. Beispielsweise erwähnt seien Substanzen für die sich die folgenden durchschnittlichen Zusammensetzungen ergeben:

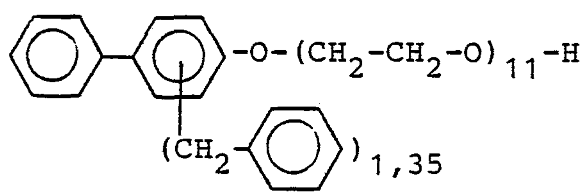

und

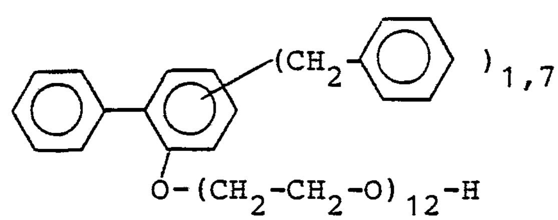

Die Alkylaryl-polyglykolether der Formel (III) sind ebenfalls bekannt.

Als organische Lösungsmittel, die in den erfindungsgemäßen Mikroemulsionen gegebenenfalls enthalten sein

können, kommen alle üblichen, mit Wasser wenig mischbaren organischen Solventien in Frage. Vorzugsweise genannt seien aromatische Kohlenwasserstoffe, wie Xylol, Toluol und Dimethyl-naphthalin, ferner chlorierte aromatische Kohlenwasserstoffe, wie Chlorbenzole, außerdem aliphatische Kohlenwasserstoffe, wie Benzin und Petrolether, weiterhin halogenierte aliphatische Kohlenwasserstoffe, wie Methylenchlorid und Chloroform, darüber hinaus cycloaliphatische Kohlenwasserstoffe, wie Cyclohexan, außerdem Alkohole und Ketone, wie n-Butanol, n-Hexanol, iso-Hexanol, n-Octanol, Cyclohexanol, Benzylalkohol, Di-n-butyl-keton und Isophoron, und ferner Ether und Ester, wie Glykolmonomethylether und Glykolmonomethyletheracetat und ferner auch Triethylphosphat.

Als Solubilisatoren, die in den erfindungsgemäßen Mikroemulsionen enthalten sein können, kommen alle üblichen Lösungsvermittler in Betracht. Vorzugsweise verwendbar sind Alkylphenole oder Kresole, die pro Mol mit 1 bis 8 Mol Ethylenoxid kondensiert sind. Speziell genannt sei in diesem Zusammenhang p-Kresol, das pro Mol mit 1 bis 8 Mol Ethylenoxid kondensiert ist.

Als Zusatzstoffe, die in den erfindungsgemäßen Mikroemulsionen gegebenenfalls enthalten sein können, kommen Konservierungsmittel, Farbstoffe, Kältestabilisatoren und Synergisten in Betracht.

Als Beispiele für Konservierungsmittel seien 2-Hydroxybiphenyl und Sorbinsäure genannt. Als Beispiele für Farbstoffe seien Azofarbstoffe und Phthalocyanin-Farbstoffe angeführt. Als Beispiele für Kältestabilisatoren seien Harnstoff, Zucker und Salze, wie Ammoniumsulfat und Natriumoleat genannt. Als Beispiel für einen Synergisten sei 3,4-Methylendioxy-6-propyl-benzyl-n-butyl-diethylenglykolether (Piperonylbutoxid) genannt.

In den erfindungsgemäßen Mikroemulsionen können die prozentualen Anteile der enthaltenen Komponenten innerhalb bestimmter Bereiche variiert werden. Der Anteil an Wirkstoff bzw. Wirkstoffen liegt im allgemeinen zwischen 0,1 und 80 Gew.-%, vorzugsweise zwischen 5 und 80 Gew.-%. Der Anteil an Emulgator-Gemisch beträgt im allgemeinen 1 bis 20 Gew.-%, vorzugsweise 3 bis 16 Gew.-%, wobei das Verhältnis der Emulgatoren zueinander ebenfalls in einem bestimmten Bereich variiert werden kann. Im allgemeinen entfallen auf 1 Teil an Emulgator der Formel (I) 0,2 bis 1,2, vorzugsweise 0,4 bis 1 Teil an Emulgator der Formel (II). Ferner entfallen auf 1 Teil Emulgator der Formel (III) im allgemeinen 0,1 bis 1,2 Teile, vorzugsweise 0,2 bis 1,0 Teile an Emulgator der Formel (II).

Mit Wasser wenig mischbare organische Lösungsmittel und/oder Solubilisatoren können in Anteilen von 1 bis 30 Gew.-%, vorzugsweise 5 bis 20 Gew.-% enthalten sein. Zusatzstoffe können in Anteilen von 0,05 bis 15 Gew.-%, vorzugsweise 0,1 bis 10 Gew.-% enthalten sein. Der prozentuale Anteil an Wasser in den erfindungsgemäßen Mikroemulsionen errechnet sich jeweils als Differenz aus 100 Gew.-% und der Summe der prozentualen Anteile der übrigen Komponenten.

In den erfindungsgemäßen Mikroemulsionen kann das Verhältnis von Wirkstoff(en) gegebenenfalls im Gemisch mit organischen Solventien und/oder Solubilisatoren einerseits zu Emulgatorgemisch andererseits in einem bestimmten Bereich variiert werden. Im allgemeinen entfallen auf 1 Teil Emulgatorgemisch 1 bis 15 Gew.-Teile, vorzugsweise 2 bis 10 Gew.-Teile an Wirkstoff(en) gegebenenfalls im Gemisch mit organischen Solventien und/oder Solubilisatoren.

Bei der Herstellung der erfindungsgemäßen Mikroemulsionen können vorzugsweise alle diejenigen Komponenten verwendet werden, die bereits im Zusammenhang mit der Beschreibung der erfindungsgemäßen Mikroemulsionen vorzugsweise genannt wurden.

Verwendet man bei dem erfindungsgemäßen Verfahren einen Wirkstoff, der bei Temperaturen bis zu 40°C im flüssigen Zustand vorliegt, so erübrigt sich im allgemeinen die Zugabe eines mit Wasser wenig mischbaren organischen Lösungsmittels und/oder eines Solubilisators.

Verwendet man bei dem erfindungsgemäßen Verfahren hingegen einen Wirkstoff, der bei Temperaturen bis zu 40°C im festen Zustand vorliegt, so ist es erforderlich, den betreffenden Wirkstoff vor der Emulgierung in einem mit Wasser wenig mischbaren organischen Lösungsmitel und/oder Solubilisator zu lösen. Die Menge an organischem Solvens und/oder Solubilisator wird dabei so bemessen, daß sie gerade zur Lösung der Festsubstanz ausreicht.

Die Reaktionstemperaturen können bei dem erfindungsgemäßen Verfahren innerhalb eines größeren Bereiches variiert werden. Im allgemeinen arbeitet man bei Temperaturen zwischen 10°C und 80°C, vorzugsweise zwischen 20°C und 60°C.

Bei der Durchführung des erfindungsgemäßen Verfahrens geht man im allgemeinen so vor, daß man zunächst eine homogene Lösung aus einem oder mehreren Wirkstoffen, Emulgatorgemisch, gegebenenfalls mit Wasser wenig mischbarem organischen Lösungsmittel und/oder Solubilisator sowie gegebenenfalls Zusatzstoffen herstellt und diese Mischung dann unter Rühren in gegebenenfalls Zusatzstoffe enthaltendes Wasser gibt. Die Mengen der Komponenten werden dabei so gewählt, daß die Komponenten in der resultierenden Mikroemulsion in der jeweils gewünschten Konzentration vorliegen. Die Reihenfolge, in der die Komponenten der organischen Phase zusammengegeben werden ist variabel. Die Zugabe der organischen Phase zu der wäßrigen Phase erfolgt zweckmäßigerweise langsam unter gleichmäßigem Rühren mit üblichen Rührgeräten. Dabei bildet sich eine feinteilige, transparente Mikroemulsion, die sich optisch von einer Lösung nicht mehr unterscheidet.

Die erfindungsgemäßen Mikroemulsionen können entweder in der zubereiteten Form oder nach vorheriger Verdünnung appliziert werden. Die Aufwandmenge richtet sich dabei nach der Konzentration der Wirkstoffe in der Mikroemulsion und nach der jeweiligen Indikation.

Die Anwendung der erfindungsgemäßen Mikroemulsionen erfolgt nach den üblichen Methoden, also zum Beispiel durch Spritzen, Sprühen oder Gießen.

Die Herstellung der erfindungsgemäßen Mikroemulsionen geht aus den folgenden Beispielen hervor.

Herstellungsbeispiele

Beispiel 1

70 g des insektiziden Wirkstoffes O,O-Dimethyl-O-(4-methylmercapto-3-methyl-phenyl)-thionophosphorsäureester werden unter Rühren bei Temperaturen zwischen 20°C und 40°C mit 8 g eines Emulgator-Gemisches versetzt, das

- zu 6 Gewichtsteilen aus einem Nonyl-phenol-polyglykolether besteht, welcher durchschnittlich 27 Oxyethylen- und 27 Oxypropylen-Einheiten pro Molekül aufweist,

und

- zu 4 Gewichtsteilen aus dem 4-(n-Dodecyl)-phenylsulfonsäure Calciumsalz (gelöst in n-Butanol)

besteht. Die dabei entstehende homogene Lösung wird innerhalb von 2 Minuten unter intensivem Rühren in 22 g entmineralisiertes Wasser gegeben. Nach beendeter Zugabe wird noch 5 Minuten nachgerührt. Es bildet sich eine transparente Mikroemulsion, die auch nach wochenlanger Lagerung bei erhöhter Temperatur keine physikalischen oder chemischen Veränderungen zeigt.

Beispiel 2

50 g O,O-Dimethyl-O-(4-methylmercapto-3-methyl-phenyl)-thionophosphorsäureester werden unter Rühren bei Temperaturen zwischen 20 und 40°C mit 14 g eines Emulgatorgemisches versetzt, das

- zu 10 Gewichtsteilen aus einem Alkylarylpolyglykolether besteht, dessen durchschnittliche Zusammensetzung aus der folgenden Formel hervorgeht

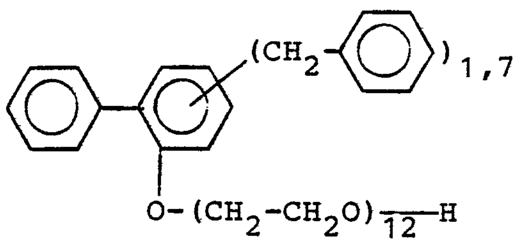

,

und

- zu 4 Gewichtsteilen aus dem 4-(n-Dodecyl)-phenylsulfonsäure Calciumsalz (gelöst in n-Butanol)

besteht. Die dabei entstehende homogene Lösung wird innerhalb von 2 Minuten unter intensivem Rühren in 36 g entmineralisiertes Wasser gegeben. Nach beendeter Zugabe wird noch 5 Minuten nachgerührt. Es bildet sich eine transparente Mikroemulsion, die auch nach wochenlanger Lagerung bei erhöhter Temperatur keine physikalischen oder chemischen Veränderungen zeigt.

Patentansprüche

1. Wäßrige Mikroemulsionen, gekennzeichnet durch einen Gehalt von

- 0,1 bis 80 Gew.-% mindestens eines in Wasser wenig löslichen agrochemischen Wirkstoffes, eines Wirkstoffes zur Bekämpfung von Schädlingen im Haushalts- und Hygiene-Bereich und/oder eines pharmakologisch wirksamen Stoffes,

- 1 bis 20 Gew.-% eines Emulgatorgemisches aus

a) mindestens einem Alkylaryl-polyglykolether der Formel

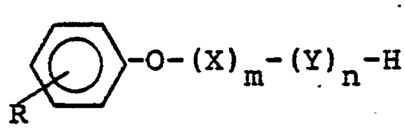

(I)

in welcher

R für Alkyl mit 8 bis 20 Kohlenstoffatomen steht,

X und Y jeweils für eine $-CH_2-CH_2-O-$, $-CH_2-\underset{CH_3}{CH}-O-$ oder $-\underset{CH_3}{CH}-CH_2-O-$Gruppe stehen, wobei jedoch X und Y nicht gleichzeitig für eine Oxyethylen- oder Oxypropylen-Einheit stehen,

m für Zahlen von 10 bis 45 steht und

n für Zahlen von 10 bis 45 steht,

und

mindestens einem Alkylarylsulfonsäure-Salz der Formel

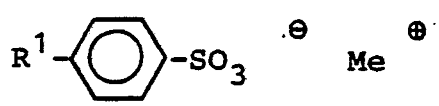 (II)

in welcher

$R^1$ für Alkyl mit 8 bis 35 Kohlenstoffatomen steht und

$Me^{\oplus}$ für ein Alkalimetallkation, ein Äquivalent eines Erdalkalimetallkations oder für ein Kation der Formel

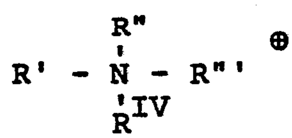

steht, worin

R', R", R"' und $R^{IV}$ unabhängig voneinander für Wasserstoff, Alkyl

mit 1 bis 4 Kohlenstoffatomen oder Hydroxyalkyl mit 1 bis 4 Kohlenstoffatomen stehen,

oder

b) mindestens einem Alkylarylpolyglykolether der Formel

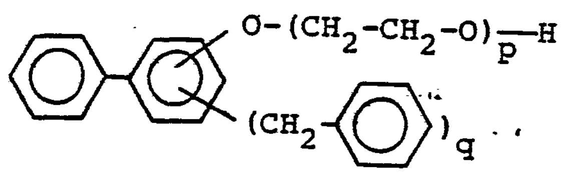

(III)

in welcher

p für Zahlen von 5 bis 20 steht und

q für Zahlen von 1 bis 3 steht,

und mindestens einem Alkylarylsulfonsäure-Salz der Formel

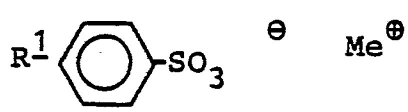

(II)

in welcher

$R^1$ und $Me^{\oplus}$ die oben angegebene Bedeutung haben,

- sowie Wasser und

- gegebenenfalls 1 bis 30 Gew.-% mindestens eines mit Wasser wenig mischbaren organischen Lösungsmittels und/oder eines Solubilisators sowie

- gegebenenfalls 0,05 bis 15 Gew.-% an Zusatzstoffen,

wobei die Summe der Komponenten jeweils 100 Gew.-% beträgt.

2. Verfahren zur Herstellung von wäßrigen Mikroemulsionen gemäß Anspruch 1, dadurch gekennzeichnet, daß man eine homogene Mischung aus

- mindestens einem in Wasser wenig löslichen agrochemischen Wirkstoff, einem Wirkstoff zur Bekämpfung von Schädlingen im Haushalts- und Hygiene-Bereich und/oder einem pharmakologisch wirksamen Stoff,

- einem Emulgatorgemisch aus

  a) mindestens einem Alkylaryl-polyglykolether der Formel

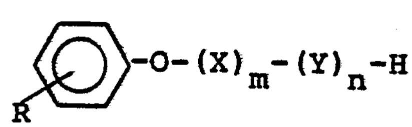

(I)

in welcher

R, X, Y, m und n die oben angegebene Bedeutung haben,

und

mindestens einem Alkylarylsulfonsäure-Salz der Formel

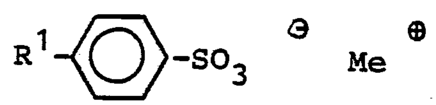 (II)

in welcher

$R^1$ und $Me^{\oplus}$ die oben angegebene Bedeutung haben,

oder

b) mindestens einem Alkylaryl-polyglykolether der Formel

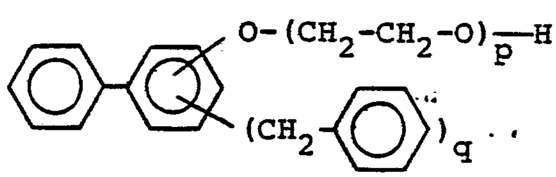 (III)

in welcher

p und q die oben angegebene Bedeutung haben

und

mindestens einem Alkylaryl-sulfonsäure-Salz der Formel

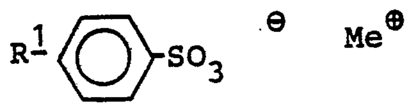

(II)

in welcher

$R^1$ und $Me^{\oplus}$ die oben angegebene Bedeutung haben,

- gegebenenfalls mindestens einem mit Wasser wenig mischbaren organischen Lösungsmittel und/oder einem Solubilisator sowie

- gegebenenfalls Zusatzstoffen

unter Rühren in gegebenenfalls Zusatzstoffe enthaltendes Wasser gibt.

3. Verwendung von wäßrigen Mikroemulsionen gemäß Anspruch 1 in der Landwirtschaft und im Gartenbau, im Haushalts- und Hygiene-Bereich und/oder im medizinischen Bereich.